(19) Europäisches Patentamt
European Patent Office
Office européen des brevets

(11) Veröffentlichungsnummer: 0 164 587
A2

(12) EUROPÄISCHE PATENTANMELDUNG

(21) Anmeldenummer: 85105797.6

(22) Anmeldetag: 11.05.85

(51) Int. Cl.⁴: A 61 K 31/44

(30) Priorität: 23.05.84 DE 3419129

(43) Veröffentlichungstag der Anmeldung: 18.12.85 Patentblatt 85/51

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Posanski, Ulrich, Dr.
Ahornweg 65
D-5064 Rösrath(DE)

(54) **Nifedipinpräparate und Verfahren zu ihrer Herstellung.**

(57) Die Erfindung betrifft neue Nifedipin-Präparate in fester Form mit verbesserter Bioverfügbarkeit, enthaltend Nifedipin und gut lösliche Füllstoffe, Verfahren zu ihrer Herstellung und ihre Verwendung als Arzneimittel, insbesondere als Coronar- und Blutdruckmittel.

EP 0 164 587 A2

Croydon Printing Company Ltd

0164587

BAYER AKTIENGESELLSCHAFT
Konzernverwaltung RP
Patentabteilung

5090 Leverkusen, Bayerwerk
V (Pha)
KS/bo/c

## Nifedipinpräparate und Verfahren zu ihrer Herstellung

Die Erfindung betrifft neue Nifedipin-Präparate in fester Form mit verbesserter Bioverfügbarkeit, enthaltend Nifedipin und gut lösliche Füllstoffe, Verfahren zu ihrer Herstellung und ihre Verwendung als Arzneimittel, insbesondere als Coronar- und Blutdruckmittel.

Es ist bereits bekannt geworden, daß Nifedipin sehr starke kreislaufbeeinflussende Wirkungen besitzt (vgl. britisches Patent 1 173 862). Aufgrund der schweren Löslichkeit von Nifedipin treten bei der galenischen Zubereitung von Arzneispezialitäten eine Reihe von Schwierigkeiten auf, wie aus zahlreichen Patenten und Patentanmeldungen für spezielle Formulierungen dieses Wirkstoffes ersichtlich wird. So betrifft z.B. das US-Patent 3 784 684 spezielle Nifedipin enthaltende Gelatinebeißkapseln, durch welche die Coronarwirkung von Nifedipin vorteilhaft genutzt werden kann. In dem britischen Patent 1 456 618 werden weiterhin feste Arzneizubereitungen beschrieben und beansprucht, welche ebenfalls eine gute Bioverfügbarkeit von

Nifedipin gewährleisten sollen. Auch in der DT-OS 2 822 882 werden feste Arzneiformen beschrieben, bei denen durch den Einsatz bestimmter Lösungsvermittler und oberflächenaktiver Substanzen die Schwerlöslichkeit des Wirkstoffs kompensiert werden soll. Auch in der europäischen Offenlegungsschrift 1 247 soll die Resorbierbarkeit von Dihydropyridinen durch die Verwendung von Polyethylenglykol und bestimmter poröser Trägersubstanzen verbessert werden.

In der Literatur werden ebenfalls verschiedene Methoden zur Verbesserung der Bioverfügbarkeit, die unmittelbar mit einer verbesserten Löslichkeit verbunden ist, beschrieben. Neben der Änderung der chemischen Struktur, z.B. durch Einbau von löslichkeitsverbessernden Substituentengruppen sind zahlreiche Methoden beschrieben, die eine Änderung der physikalischen Eigenschaften des Wirkstoffesbetreffen. So wird z.B. die Zerkleinerung von Wirkstoffpartikeln, Veränderung der Kristallstruktur, die Herstellung von Salzformen, Zugabe von Netzmitteln, Komplexbildung mit anderen Substanzen, Verwendung von biologischen Carriern, Erstellung fester Dispersionen (Copräzipitate) oder die Herstellung von Oberflächenadsorbaten vorgeschlagen (vgl. S.H. Yalkowskay, Drugs and the pharmaceutical science, 12, 135-180 (1981) und J. Polderman, Formulation and preparation of dosage forms, Elsevier/North-Holland Biomedical Press, 1977, 215-219).

Alle bisherigen Versuche, die schlechte Löslichkeit von Nifedipin durch bestimmte Maßnahmen zu kompensieren und gleichzeitig eine gute Bioverfügbarkeit zu gewährleisten, besitzen eine Reihe von Nachteilen. Der Einsatz von oberflächenaktiven Substanzen, Lösungsvermittlern und bestimmten Trägerstoffen, die eine besondere Oberfläche haben, z.B. porös sind, führt häufig zu Verabreichungsformen, bei denen die Präparate unerwünscht groß sind. Zur Erleichterung des Schluckens werden solche Tabletten oder Kapseln häufig in spezifische Formen wie z.B. Ellipsoide oder Längsformen überführt, was jedoch bei Präparaten mit einem Gewicht über 400 mg auch nicht immer zu befriedigenden Ergebnissen führt. Auch das häufigere Einnehmen von kleineren Präparaten stellt keine befriedigende Lösung dar.

Die vorliegende Erfindung betrifft neue feste Nifedipin-Präparate mit verbesserter Bioverfügbarkeit, enthaltend 1 Gewichtsteil Nifedipin und 1,0 bis 20, vorzugsweise 2,0 bis 15 Gewichtsteile eines gut wasserlöslichen Füllstoffes und gegebenenfalls weitere übliche Hilfs- und Trägerstoffe.

Von besonderem Interesse sind erfindungsgemäße Nifedipin-Präparate, die als gut wasserlösliche Füllstoffe Zucker und/oder Zuckeralkohole, wie z. B. Laktose, Saccharose, Fructose, Glucose, Mannit, Sorbit und/oder Salze wie Magnesium-, Calcium-, Kalium-, Natrium-, Ammonium-Salze, wobei das Anion z. B. Chlorid, Carbonat, Citrat, Tartrat, Lactat, Acetat, Hydrogencarbonat sein kann, enthalten.

Als gut wasserlösliche Füllstoffe gelten alle physiologisch unbedenklichen Stoffe, deren Löslichkeit mindestens

15 g pro 100 ml Wasser, insbesondere mindestens 20 g pro 100 ml Wasser bei 25°C beträgt.

Insbesondere seien Mannit, Lactose, Natriumchlorid und Kaliumcitrat genannt.

Die Herstellung der erfindungsgemäßen Präparate erfolgt, indem man Nifedipin und den leicht wasserlöslichen Füllstoff gemeinsam granuliert und gegebenenfalls zu Tabletten verpreßt.

Die Herstellung des erfindungsgemäßen verwendeten Nifedipins erfolgt vorzugsweise durch Umsetzung einer Ylidenverbindung der Formel II

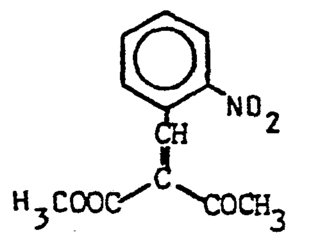

(II)

mit einer Enaminverbindung der allgemeinen Formel III

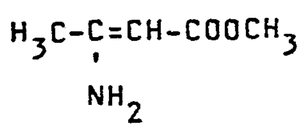

(III)

in niederen Alkoholen mit 1 bis 4 Kohlenstoffatomen bei Temperaturen zwischen 40 und 100°C.

Eine bevorzugte Verfahrensvariante besteht darin, daß Reaktionswasser durch azeotrope Destillation zu entfernen. Die Verbindungen II und III können entweder in molaren Mengen oder mit einem geringen Überschuß der Enaminverbindung III eingesetzt werden.

Die Herstellung der Ylidenverbindung II erfolgt durch Reaktion molarer Mengen eines Ketocarbonsäureesters mit o-Nitrobenzaldehyd in Gegenwart von niederen Alkoholen mit 1 bis 4 C-Atomen als Lösungsmittel und in Gegenwart von katalytischen Mengen Piperidinacetat bei Temperaturen zwischen 20 und 60°C. Die Enaminverbindung der Formel III wird hergestellt durch Umsetzung eines Ketocarbonsäureesters mit Ammoniak in einem niederen Alkohol (1-4 C-Atome), vorzugsweise in Isopropanol, bei Temperaturen zwischen 0 und 40°C. Der Reaktionspartner Ammoniak kann auch in wäßriger Lösung eingesetzt werden.

Bei Kenntnis des Standes der Technik ist es ausgesprochen überraschend, daß durch die gemeinsame Granulation von Nifedipin mit einem der genannten leicht löslichen Füllstoffe eine signifikante Erhöhung der Freisetzung und damit eine signifikante Verbesserung der Bioverfügbarkeit für Nifedipin erreicht werden kann. Dieser Effekt ist nicht abhängig von der Anwesenheit zusätzlicher Hilfsstoffe, die nach Kenntnis des Standes der Technik die Freisetzungsrate erhöhen wie z.B. Komplexbildnern oder oberflächenaktiven Stoffen.

Im Freisetzungstest nach der USP-Paddle-Methode unter den folgenden Bedingungen

900 ml 0,1 N-Salzsäure

Paddle-Drehzahl 100 U/Min.

wurden nach 1 Stunde Freisetzungsraten für Nifedipin von 100 % erreicht für erfindungsgemäße Präparate (Produkt gemäß Beispiel 1). Eine entsprechende Zubereitung einer herkömmlichen Nifedipintablette, in welcher das Nifedipin in gleicher kristalliner Zusammensetzung

eingesetzt wurde und die die gleichen Hilfsstoffe enthält, zeigt nach 1 Stunde nur eine Freisetzungsrate von 43 %. Der Verlauf der Freisetzung ist aus der Tabelle 1 ersichtlich.

Tabelle 1

In vitro - Freisetzung von nifedipinhaltigen Tabletten (USP-Paddle-Methode)

| | kumulative Freisetzung von Nifedipin in Prozent der eingesetzten Dosis | |
|---|---|---|
| Freisetzungszeit [h] | Tablette Beispiel 1 | Tablette Vergleichsbeispiel |
| 0,25 | 95 % | 18 % |
| 0,5 | 100 % | 30 % |
| 1 | 100 % | 43 % |

Vergleichsbeispiel

100 g Nifedipin, 2080 g Maisstärke, 1150 g Cellulosepulver und 50 g Milchzucker werden in einem Planetenmischer 5 Minuten gemischt und mit einer wäßrigen Polyvinylpyrrolidon/Tween 80-Lösung (Feststoffgehalt 200 g/5 g) 15 Minuten granuliert. Die feuchte Masse wird durch eine Raspel (2,0 mm Ø Siebeinsatz) gegeben und in einem Wirbelschichttrockner auf einen Endwassergehalt von ca. 5 % getrocknet.

Das getrocknete Granulat wird durch Siebung (1,0 mm-Siebmaschenweite) egalisiert und intensiv mit 15 g Magnesiumstearat vermischt. Anschließend wird das Granulat zu 360 mg schweren Tabletten verpreßt.

Ausführungsbeispiele:

Beispiel 1

100 g Nifedipin, 1580 g Maisstärke, 1400 g Milchzucker und 300 g Cellulosepulver werden in einem Planetenmischer 5 Minuten gemischt und mit einer wäßrigen Polvinylpyrrolidon/Tween 80-Lösung (Feststoffgehalt 200 g/5 g) 15 Minuten granuliert. Die feuchte Masse wird durch eine Raspel (2,0 mm Ø-Siebeinsatz) gegeben und in einem Wirbelschichttrockner auf einen Endwassergehalt von ca. 4,5 % getrocknet. Das getrocknete Granulat wird durch Siebung (1,0 mm-Siebmaschenweite) egalisiert und intensiv mit 15 g Magnesiumstearat vermischt. Anschließend wird das Granulat zu 360 mg schweren Tabletten verpreßt. Die Tabletten können mit einem Schutzlack überzogen werden.

Beispiel 2

200 g Nifedipin, 3160 g Maisstärke, 1800 g Milchzucker und 700 g Cellulosepulver werden in einem Wirbelschichtgranulator (Glatt WSG 5) erwärmt und zunächst mit einer wäßrigen Natriumlaurylsulfatlösung (Gehalt 10 g) besprüht. Danach wird das Pulver mit einer wäßrigen Hydroxypropylmethylcellulose-Lösung (Gehalt 400 g) granuliert und auf eine Endfeuchte von ca. 4,5 % getrocknet, abgesiebt (Siebweite 0,8 mm Ø) und in einem Kubusmischer mit 20 g Magnesiumstearat vermischt. Das preßfertige Granulat kann zu 314,5 mg schweren Tabletten verarbeitet und in einer Accela-Coater mit einer lichtundurchlässigen Hülle überzogen.

Beispiel 3

1000 g Mannit, 400 g Nifedipin, 600 g Cellulosepulver, 1200 g Maisstärke, 1200 g Magnesiumcarbonat und 40 g Natriumlaurylsulfat werden in einem Mischgranulator (z.B. Lödige MGT 30) 1 Minute gemischt und 5 Minuten lang mit Stärkekleister (Stärkegehalt 200 g) granuliert bei einer Rotordrehzahl des Granulators von 200 U/Min. Durch Zerhacker-Stufe II wird eine mittlere Teilchengröße von ca. 200 - 300 µ erhalten. Das Granulat wird über eine Raspel (Siebeinsatz 2,0 mm Ø) gegeben und in einem Fließbetttrockner (z.B. Glatt WST 5) bis auf eine Endfeuchte von ca. 4,0 % Wasser getrocknet. Das getrocknete Granulat wird mit 100 g Magnesiumstearat in einem Mischer 10 Minuten gemischt und anschließend zu Tabletten mit einem mittleren Durchmesser von 9 mm und einem Gewicht von ca. 237 mg verpreßt.

Die erhaltenen Tabletten können zusätzlich mit einer lichtundurchlässigen Lackhülle überzogen werden, die z.B. rotes Eisenoxid als Lichtschutz enthält.

Beispiel 4

500 g Lactose, 200 g Nifedipin, 450 g Cellulosepulver, 400 g Maisstärke, 650 g Calciumcarbonat, 25 g Tween 80 und 50 g Magnesiumstearat werden in einem Kubusmischer gemischt und anschließend trocken kompaktiert (Alexanderwalze WP50; Kompaktierungsdruck 35 bar). Die Schülpen werden vorgebrochen, mit einem 1,25 mm-Sieb egalisiert und mit einer Mischung aus 25 g Magnesiumstearat und 200 g Maisstärke homogen vermischt. Das fertige Granulat wird zu 250 mg schweren Tabletten mit 9 mm Ø verpreßt.

Die erhaltenen Tabletten können direkt verwendet werden oder alternativ noch einen Schutzlack, bestehend aus Hydroxypropylmethylcellulose, Polyethylenglykol und Eisenoxiden oder anderen physiologisch verträglichen Farbpigmenten oder Farblacken erhalten.

Beispiel 5

750 g Mannit, 300 g Nifedipin, 855 g Maisstärke, 975 g Magnesiumcarbonat, 150 g vorverkleisterte Maisstärke und 600 g Cellulosepulver werden 8 Minuten in einem Planetenmischer (Collette MP 20) gemischt und 15 Minuten lang, nach kontinuierlicher Zugabe von 250 ml einer 2,0 %igen, wäßrigen Natriumlaurylsulfatlösung, bei erhöhter Drehzahl des Mischers granuliert. Die feuchte Masse wird geraspelt, in einem Wirbelschichttrockner auf ca. 3,8 % Endfeuchte getrocknet und durch Siebung (1,0 mm Maschenweite) egalisiert. Die Endmischung wird nach Untermischung von 75 g Magnesiumstearat erhalten und zu 247 mg schweren Tabletten verpreßt.

Die erhaltenen Tabletten können zusätzlich mit einer tageslichtundurchlässigen Hülle überzogen werden. Das Granulat kann alternativ in Hartgelatinekapseln abgefüllt werden.

Patentansprüche

1. Festes Nifedipin-Präparat mit verbesserter Bioverfügbarkeit, enthaltend 1 Gewichtsteil Nifedipin und 1,0 bis 20 Gewichtsteile eines leicht wasserlöslichen Füllstoffes und gegebenenfalls übliche Hilfs- und Trägerstoffe.

2. Nifedipin-Präparat gemäß Anspruch 1, enthaltend 2 bis 15 Gewichtsteile eines leicht wasserlöslichen Füllstoffes.

3. Nifedipin-Präparat gemäß den Ansprüchen 1 bis 2, enthaltend Lactose und/oder Mannit als wasserlöslichen Füllstoff.

4. Nifedipin-Präparat gemäß den Ansprüchen 1 bis 3 in Form von Granulat.

5. Nifedipin-Präparat gemäß den Ansprüchen 1 bis 3 in Form von Tabletten, Lacktabletten oder Dragees.

6. Verfahren zur Herstellung von Nifedipin-Präparaten gemäß Anspruch 1, dadurch gekennzeichnet, daß man 1 Gewichtsteil Nifedipin und 1,0 bis 20 Gewichsteile eines leicht wasserlöslichen Füllstoffes gemeinsam mit Hilfs- und Trägerstoffen granuliert und gegebenenfalls dieses Granulat zu üblichen Applikationsformen weiterverarbeitet.

7. Verfahren gemäß Anspruch 6, dadurch gekennzeichnet, daß man 1 Gewichtsteil Nifedipin gemeinsam mit 2 bis 15 Gewichtsteilen eines leicht löslichen Füllstoffes mit Stärke, Cellulose und gegebenenfalls sonstigen üblichen Hilfsstoffen in einem Granulator wäßrig granuliert und das erhaltene Granulat ebenfalls in eine übliche Applikationsform überführt.

8. Verfahren zur Herstellung von Präparaten gemäß Anspruch 1, dadurch gekennzeichnet, daß man die Ylidenverbindung der Formel II

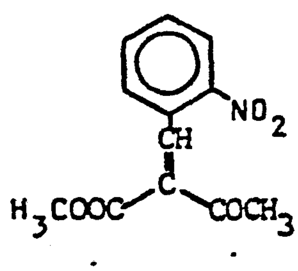

(II)

mit der Enaminverbindung der Formel III

$$H_3C-\underset{NH_2}{C}=CH-COOCH_3 \qquad (III)$$

in Gegenwart von niederen Alkoholen (1-4 C-Atome), vorzugsweise Isopropanol, als Lösungsmittel bei Temperaturen zwischen 0 und 40°C umsetzt und das so erhaltene Nifedipin gemäß dem Anspruch 6 in eine geeignete Applikationsform überführt.

9. Verwendung von Nifedipin-Präparaten gemäß Anspruch 1 bei der Bekämpfung von Kreislauferkrankungen.